# EUROPEAN PATENT APPLICATION

(11) **EP 3 862 572 A1**
(43) Date of publication of application: **11.08.2021**
(21) Application number: 18936222.1
(22) Date of filing: 05.10.2018
(51) Int. Cl.: F04D 29/38, A61M 16/00

(54) **IMPELLER DEVICE FOR PROVIDING ASSISTED VENTILATION**

(71) Applicant: Técnicas Biomédicas para la Salud, S.L., 08930 Sant Adrià del Besòs (ES)
(72) Inventor: CARBO BECH, Albert, 08930 Sant Adria Del Besos (Barcelona) (ES); DURAN PRAT, Jordi, 08930 Sant Adria Del Besos (Barcelona) (ES); ANCHUELA ARNALTE, Carlos, 08930 Sant Adria Del Besos (Barcelona) (ES); LÓPEZ RODRIGUEZ, Juan, Luis, 08930 Sant Adria Del Besos (Barcelona) (ES); CRESPO CABRERA, Jordi, 08930 Sant Adria Del Besos (Barcelona) (ES); PETIT MEROÑO, Joan, Ramon, 08930 Sant Adria Del Besos (Barcelona) (ES); FONTANALS GARCIA, Alfred, 08930 SANT ADRIA DEL BESOS (Barcelona) (ES); PALOU FUSTÉ, Jaume, 08930 Sant Adria Del Besos (Barcelona) (ES)
(74) Representative: Herrero & Asociados, S.L.
(86) International application number: PCT/ES2018/070648
(87) International publication number: WO 2020/070349

(57) **Abstract**

The driving device for providing assisted ventilation comprises an air inlet (15), an impeller (7) provided with blades (8) rotatably driven by a motor (6), and an air outlet (5), the rotation of said impeller (7) being caused by the air circulation through a duct (4) from the air inlet (15) to the air outlet (5), wherein said blades (8) of the impeller (7) are flexible, vibrating at least one of its ends by the thrust caused by said circulating air.

## Description

The present invention refers to a driving device for providing assisted ventilation, comprising noise reduction means, thus improving user comfort.

### Background of the invention

Sound, which consists of waves of fluid pressure variation, normally originates when a fluid collides and experiences a pressure variation against at least one solid surface or another fluid, the surface being in motion relative to the fluid.

The transmission of sound is a bidirectional process, that is, the pressure variation waves whose energy is proportional, among other things, to the density of the fluid and the amplitude of the vibration, by exerting a force on the different points of a solid surface, make it vibrate and behave like another secondary sound source. Therefore, to reduce secondary sound, vibrations from the solid surface have to be absorbed, and sound energy can also be reduced by reducing the density of the fluid.

Regarding vibrations, these are transmitted worse in materials that have preferably viscous elasticities. For this, damping elements are added to hold or couple the vibrating elements and limit the transmission of vibrations to more noise-emitting surfaces.

Active noise cancellation systems are based on the generation of push-pull waves with respect to the noise present in the area of the space where it is desired to be eliminated, highlighting the fact that, for physical reasons, the position of the microphone that captures the sound, or noise sensor, and that of the speaker that emits the cancellation waves do not coincide, so there is a delay and secondary bounces, between the captured wave and the wave that passes in front of the cancellation speaker.

Therefore, the phase and amplitude of each frequency component of the wave that cancels the unwanted noise has to be calculated for each of the frequency components, so the cancellation loop varies as a function of the shape and nature of the noise to be eliminated and of the shape of the cancellation acoustic channel.

In this way, efficient noise cancellation does not simply consist of a phase change or inversion of the measured signal and an adjustment of its amplitude, except in the case that the unwanted noise has a pure and stationary tone nature, in which case, this method is applicable, but it is an ideal and rare case.

For this reason, adaptive and reiterative mechanisms have been suggested that approximate the wave generated by the cancellation system successively to an optimal elimination of the signal based on mathematical algorithms, such as, for example, FxLMS, which is based on finding the minimum mean quadratic of the difference between a synthetic signal and the residual signal at the point where you want to eliminate noise.

This family of algorithms based on mathematical reiteration systems from previous sound readings, modifies a series of coefficients in memory until reaching a mathematical optimum where convergence occurs (where the error tends to 0) and they behave very efficiently for signals of the quasi-stationary type and with pure frequency components, where the frequency pattern of the noise varies in a smooth way.

These noise cancellation systems can suffer situations of non-convergence (the error does not tend to 0) of the result obtained and of slow adaptation, with the speed of adaptation and convergence apparently opposite situations.

In case of non-convergence, the noise cancellation system ends up being transformed into a noise generation system (malfunction), which maintains that state due to its own memory of coefficients and also as a consequence of the limitation of the dynamic range of the elements of the control loop.

Furthermore, for high adaptation speeds there are a greater number of low convergence problems, and vice versa, since for good convergences the adaptation speeds are slow.

In the event of slow adaptation, the system does not react quickly enough to variations in the phenomenon to be canceled, as is the specific case of turbulent noises produced by a fan and ducts of a continuous positive airway pressure (CPAP) device, which change working conditions based on an individual's breathing.

Noise cancellation systems have better performance and results, the purer, the fewer and more constant the frequencies of the sound components to be canceled.

The different noises that a ventilation system can generate are more annoying the purer and narrower the multiple waves they generate, and as a general rule in purely mechanical systems the noise spectrum is tried to be as wide as possible and with the minimum of amplitude, concentrating the spectrum in the areas with less sensitivity for the human ear.

All this suggests the introduction of mechanisms that favor mathematical convergence without impairing speed of adaptation and designing mechanical systems that, relying on the resource of active cancellation, if they end up generating noise, that it be with waves as pure and constant as possible, with the minimum of harmonics, which is the complete opposite of what is sought when there is no active noise cancellation (purely mechanical systems), so the solutions that act in the mechanical system will be linked with those that act in the system of active cancellation, when trying to improve the mathematical convergence of the assembly.

Active noise cancellation systems in ducts are especially efficient when the wavelength of the sound to be canceled is in close proximity (same order of magnitude) as the internal diameter of the duct or dimension of the cavity, in such a way that, noticeably, only one wave front is transmitted along the tube.

These cancellation ducts have the difficulty that the solid structure of the duct where the cancellation is carried out, and where the microphones and speakers are positioned, behaves as a solid vibration transmitter, from the inner air, from the speaker and towards cancellation microphones, not being properly that vibration the one that is tried to annul in the air of the tube.

All this suggests the possibility of isolating the useful air signal measured by the microphone from the "parasitic or crosstalk" signal caused by the vibration of the solid on the microphone.

Measuring the noise in the ducts where air circulates has the difficulty that the holes themselves where the signal is captured are noise generators due to the turbulence of the air at their end.

Regarding the nature of the noise generated by a fan, it should be noted that:
When having rotating element, this is a source of vibrations due to mechanical unbalance, and these vibrations will be harmonic with the frequency of rotation of a combination of one or more vectors of centrifugal force distributed perpendicularly along the axis of rotation, vectors rotating at measurable angular speed. Current balancing solutions in air driving devices do not have the possibility of balancing the assembled system, balancing the different elements that compose it separately.

By having a series of blades that rotate rapidly approaching and moving away from discrete elements of their contour, abrupt thrusts or wave fronts are also produced on the air periodically, which will be harmonics of the rotation frequency and which, being abrupt, contain a plurality of harmonics. In traditional radial fans, the main point where annoying sound occurs is in the vicinity of the apex of the outlet duct, just at the moment when the impeller blades pass in front of it as the air experiences a pressure variation as it is between two surfaces that come close to each other abruptly.

As the air moves through the ducts, either in the moving or fixed parts, especially in the surfaces that change their orientation in space with small radii, turbulence and vibrations of the type Vortex-Induced Vibration ("Vortex-Induced Vibration") that appear in the frequency spectrum within a band as small peaks that appear and disappear and whose frequency and amplitude have a variability that is a function of the Reynolds number of the fluid in motion.

The higher the Reynolds number, the greater the amplitude and variability, the carrier frequency of this type of noise being proportional to the Strouhal number (constant obtained by the shape of the solid surface) at the speed of the fluid and inversely proportional to the dimensions of the form that produces them.

Therefore, the object of the present invention is to provide a driving device for providing assisted ventilation, which includes noise reduction, means to improve the comfort of users.

### Description of the invention

With the driving device of the invention, the aforementioned drawbacks are solved, presenting other advantages that will be described below.

The driving device for providing assisted ventilation according to the present invention comprises an air inlet, an impeller provided with blades rotatably driven by a motor, and an air outlet, the rotation of said impeller causing the circulation of air through a duct from the air inlet to the air outlet, wherein said impeller blades are flexible, at least one of their ends vibrating by the thrust caused by said circulating air.

By virtue of this feature, the noise produced by the impeller is greatly reduced, which makes it possible to provide a driving device that emits less noise than similar conventional devices.

According to a preferred embodiment, said blades are curved, and preferably the end of each blade that is closest to the axis of rotation of the impeller is fixed with respect to the impeller and the end of each blade that is furthest from the axis of rotation of the impeller is the end that vibrates by the thrust caused by the circulating air.

To optimize active cancellation, the vibrations induced by the blades as an elastic system with a mass, shall be in a narrow frequency range to optimize their active cancellation and shall approach an integer multiple of the frequency of rotation of the impeller in the more usual working regimes, in such a way that the relative movement of the end of the blades is in opposition to that of the impeller itself at the moment in which they pass in front of the vertex of the outlet duct, thus smoothing the wave front caused at that moment and then totally or partially dissipating the energy accumulated by the bending of the blade in the space of time of the turn of the impeller, thereby considerably reducing the number of harmonics present.

To facilitate the balancing of the components subjected to vibrations (motor and impeller) in their work location and for preventive maintenance, the device according to the present invention can also comprise accelerometers mounted or coupled to the body where the motor is held. Furthermore, it may also comprise a motor angle gauge, which measures the angle of rotation of the impeller.

To reduce the noise caused by the air and transmitted in the solid that forms the duct and consequently the noise emitted by the device, the body that surrounds the air duct preferably comprises a plurality of internal cavities, wherein a vacuum level can be practiced, each internal cavity comprising at least one support column.

To guide the incoming air flow, reducing the level of rotational change and, therefore, the acoustic wave generated at the air inlet, said air inlet advantageously comprises a plurality of air directing vanes, which preferably have spiral shape.

To generate vibrations induced by vortices, and that are in a narrow frequency range and thus optimize their active cancellation, the duct is designed so that the Strouhal number of each shape present in an area of the duct with respect to the speed in that area, maintain a homogeneous relationship in the different areas of the air duct.

In order to reduce turbulent noise at the elbows, and also to ensure that it maintains a homogeneous Strouhal number / speed ratio, said duct may comprise a central partition, for example, placed at the end of the duct closest to the air outlet.

For said active noise cancellation, the device according to the present invention also comprises one or more microphones to measure the sound produced by the air inside the channel, said microphones being located in housings provided with a hole in communication with the channel.

According to a preferred embodiment, said hole at its end of the channel is conical or funnel-shaped and around said hole a circular groove is arranged that allows turbulence to be moved away from the collection point.

The hole can also be covered by a porous, rigid or semi-rigid structure that allows a laminar flow on its surface, therefore, without appreciably affecting the transmission to the microphone of the sound prevailing in the duct.

Furthermore, the device according to the present invention may also comprise additional microphones for detecting the sound produced by vibrations in the body of the device, said additional microphones being located in a closed housing.

To solve the cases of non-convergence of the active cancellation algorithms, an, a reset mechanism of the adaptive filter coefficients is added to active cancellation system, such as FxLMS, that recovers a preset state when excessive residual noise is detected, the choice of the set of coefficients is made by measuring the working regime of the fluid and motor system, such as flow, pressure and rotational speed, values that have been previously stored, when a convergence has been detected that is valid for a known work regime.

### Brief description of the drawings

For a better understanding of what has been stated, some drawings are attached in which, schematically and only as a non-limiting example, a practical case of embodiment is represented.
Figure 1 is a perspective view of a driving device in accordance with the present invention;
Figure 2 is a cross-sectional elevation view of the driving device of Figure 1;
Figure 3 is a cross-sectional elevation view, similar to Figure 2, showing the internal cavities for reducing air turbulence;
Figure 4 is a perspective view of the air inlet to the device, with its upper part removed to show the shape of the blades of said inlet;
Figure 5 is a sectional elevation view of the driving device according to the present invention, wherein the partition placed inside the air duct can be seen;
Figure 6 is a longitudinal section plan view of the driving device according to the present invention, wherein the impeller with its blades is shown;
Figure 7 is a sectional view of the part of the driving device according to the present invention wherein the motor and the impeller are located;
Figure 8 is a block diagram of the active noise cancellation system that includes the driving device according to the present invention;
Figure 9 is a sectional elevation view of the part of the device where the microphones are located; and
Figure 10 is a sectional perspective view of a microphone placed inside its housing.

### Description of a preferred embodiment

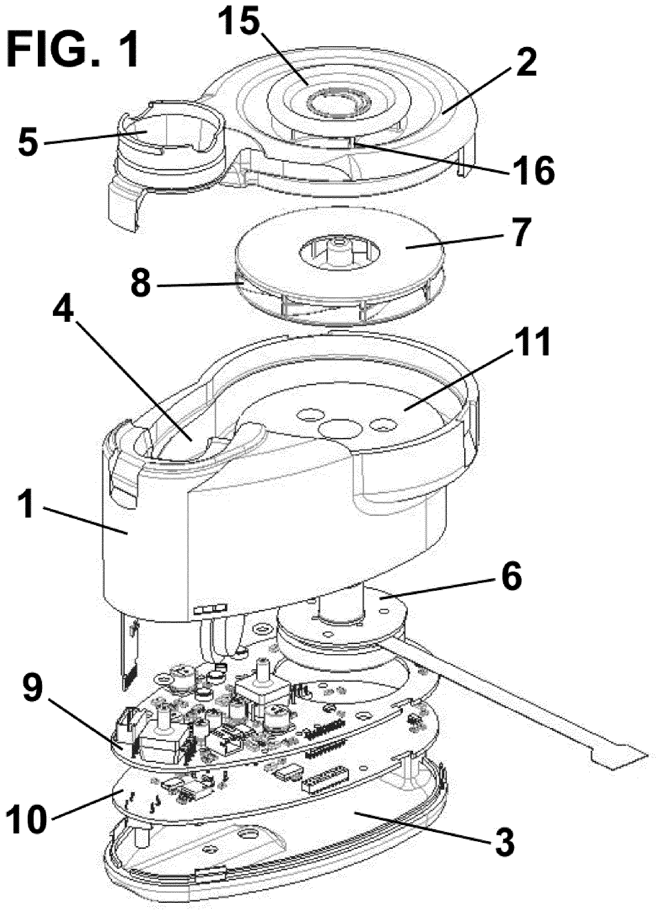

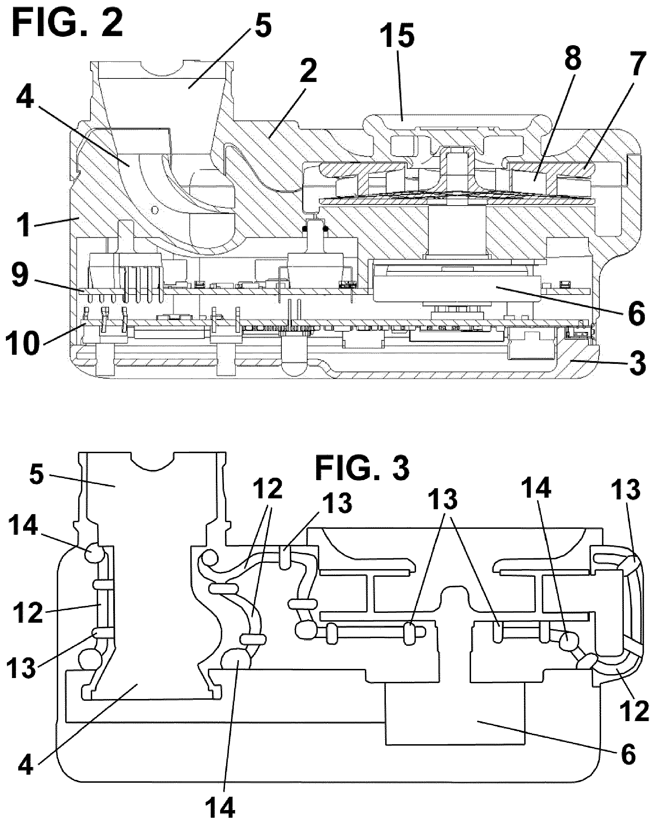

As shown in Figures 1 and 2, the driving device for providing assisted ventilation according to the present invention comprises a housing 1 provided with an upper cover 2 and a lower cover 3, said housing 1 defining inside a channel 4 for conveying air, which air is provided to a user from an inlet 15 to an outlet 5.

Furthermore, the driving device according to the present invention comprises inside a motor 6 that rotatably drives an impeller 7 provided with blades 8, the rotation of which causes air circulation.

This driving device also comprises a first and second plates 9, 10 for mounting electronic components to guarantee the correct operation of the driving device.

Part of the noise produced by this driving device is due to the rotation of its mechanical assembly, that is, the motor 6 and the impeller 7.

Consequently, to reduce noise, vibration reduction by balancing in two or more rotating planes is necessary. This balancing is carried out in three ways, by separating the motor 6, the impeller 7 and then the motor 6 and impeller 7 assembly. This balancing is carried out by adding or removing material, for example, by means of cavities.

In particular, the reduction of vibrations by balancing in one or more planes in rotation is carried out by applying successive approximations towards a more balanced system, obtaining the axial buckling and vibration components of both the motor 6 and the impeller 7.

Obtaining these components is carried out, for example, by means of two three-axis accelerometers far apart and attached to the structure that holds the motor 6. Preferably, a rotation angle gauge is also used, which measures the rotation of the motor's output shaft 6.

By solving the components of each force by linear combination of these quantities and modifying the centers of masses of the motor 6 and of the impeller 7, by adding or subtracting material, the correct balance is achieved, avoiding or reducing a source of noise.

Another source of transmission of vibrations and noise are the fasteners between the different components that make up the driving device according to the present invention.

To avoid vibrations, the different components that make up the device must be held together, minimizing the transmission of movement from moving parts to static parts.

For this, said components are fixed at the points where the vibration modes present minimum amplitude at the typical frequencies (with greater amplitude) present in the noise source at different working regimes. To know these values, interferometric measurement is used, with or without laser, sand on the surface, or numerical simulation.

Another source of noise in the driving device according to the present invention is the turbulence of the air itself when it circulates abruptly in the different points of the cavity 11 where the impeller 7 is housed, the noise being transmitted through the air towards the duct 4.

To avoid this transmission, internal cavities 12 are arranged in said duct 4, wherein a degree of vacuum will be practiced. These internal cavities are preferably arranged around the acoustic vibration sources to isolate them from the outside of the device.

In addition, to prevent the structure of the duct 4 from being crushed by air pressure, support columns 13 are added at the points where the vibration modes may have minimum amplitude at the typical frequencies (with greater amplitude) present at the noise source at different work regimes.

To know these points, interferometric measurement with or without laser, sand on the surface or numerical simulation can be used. Air jet ducts 14 can also be added, which can also collect waste from 3D printing manufacturing.

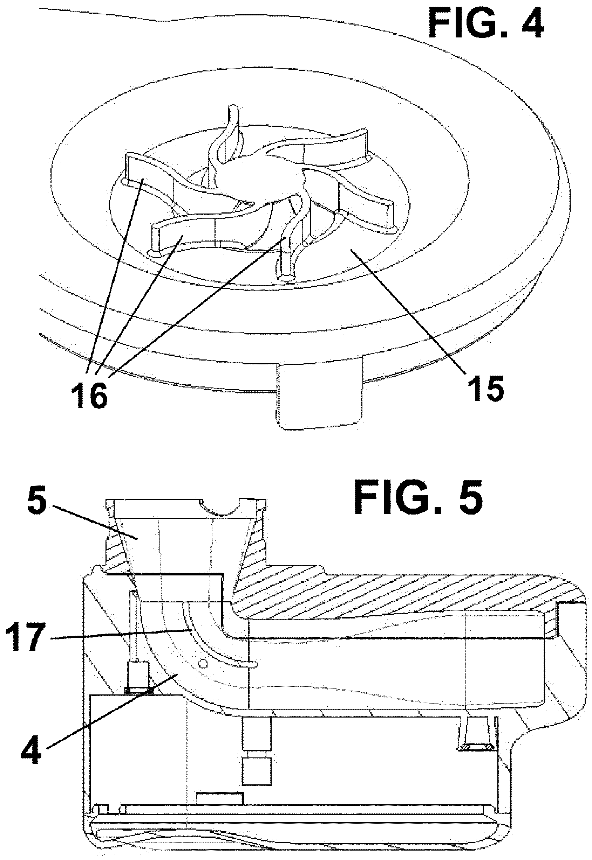

In figure 4 vanes 16 are shown in perspective and are formed in the air inlet 15 to the driving device. These vanes 16, which are static, guide the incoming air flow, reducing the level of rotational change and, therefore, the acoustic wave generated at the air inlet 15.

As can be seen in this Figure 4, each vane 16 preferably has a spiral shape, although it could have any suitable shape.

Furthermore, to favor the appearance of vortex-induced vibrations ("Vortex Induced Vibrations") in a narrow frequency range, the driving device according to the present invention also comprises a partition 17 placed inside the duct 4. As can be seen in Figure 5, said partition 17 is preferably curved and is located at the end of the duct 4 closest to the outlet 5.

The aim of vortex-induced vibrations to be in a narrow frequency range is to optimize their active cancellation, as will be described later.

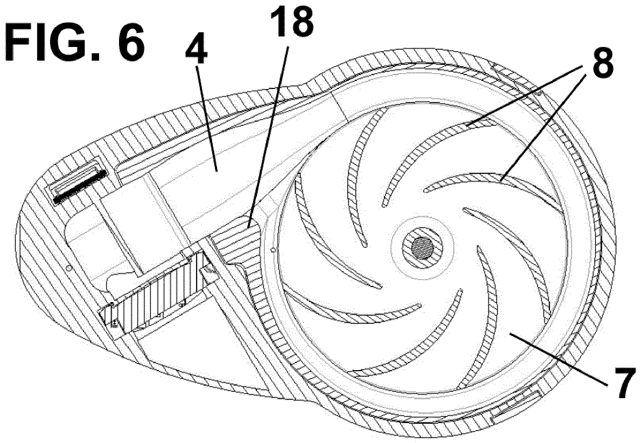

To reduce the noise generated, it is also important to smooth the wave fronts generated by the blades 8 of the impeller 7, shown in greater detail in figure 6. For this, said blades 8 are flexible, and vibrate on a push-pull basis with the wave generated reducing the noise generated.

In particular, said blades 8 are curved and their end closest to the axis of rotation of the impeller 7 is fixed, the end farthest from the axis of rotation of the impeller 7 being the one that moves by virtue of the flexible nature of the blades 8 during the vibration.

When the blades 8 of the impeller 7 vibrate with respect to the impeller 7, with respect to the speed of the blades 8 with respect to the impeller 7, there are times when:
- the speed is positive and is added to the tangential speed of the impeller 7,
- the speed is neutral and the blades 8 move at the same speed as the impeller 7, or
- the speed is negative and is subtracted from the tangential speed of the impeller.

When it is desired to silence the impeller 7, the aim is to achieve that the frequencies of oscillation of the blades 8 be multiples of the speed of rotation of the impeller 7 plus a small phase shift, not close to 180 degrees. In this way, the blades 8 reduce their tangential speed, just at the moment of passing in front of the exit vertex 18, in such a way that the transient wave generated at that moment is reduced.

The oscillation of the blades 8 is maintained by virtue of the energy they absorb in the form of bending at the moment when the air pressure rises sharply at the outlet vertex 18. This energy, instead of generating a sudden transient with a plurality of harmonics, gradually dissipates in the form of a decreasing oscillation at the typical oscillation frequency of the blades 8 and over a longer time than the sudden pressure impact would suppose, therefore it supposes a lower noise level.

The damped wave of known frequency is easily eliminated by an active noise cancellation system with the use of microphones and speakers.

Likewise, the superposition of the out-of-phase waves of each of the different blades 8 of the impeller 7 supposes a subtractive sum, which further reduces the effect of the waves generated. In this way, the frequency of rotation of the impeller 7 will be gradually adjusted until a minimum of sound emission is achieved.

To further reduce the vibrations of the vibrating components, in particular the motor 6 and the impeller 7, elastic means integrated in the holding structure of these components can be included.

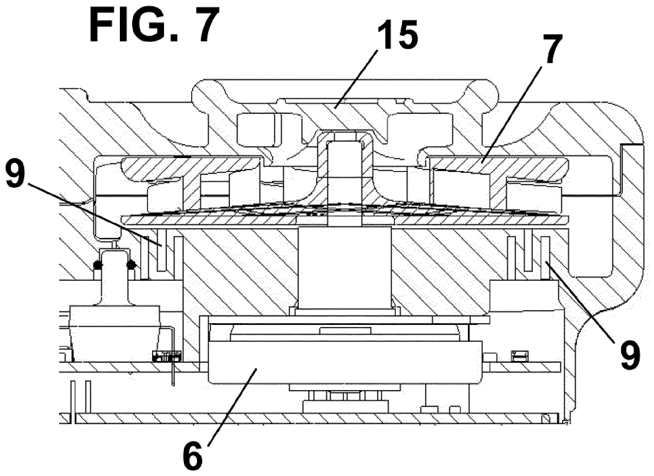

For example, as shown in figure 7, said elastic means may be formed by grooves 19 arranged alternately in the part of the housing 1 which is arranged between the motor 6 and the impeller 7.

The driving device according to the present invention also comprises an electronic active noise cancellation system, which is mounted on the first and / or second plates 9, 10.

Said electronic system improves and optimizes active noise cancellation, for example, through the use of tables of cancellation parameters or filter coefficients, indexed or calculated from the angular speed and / or the pressure and / or the flow that supplies the device, to accelerate the convergence process faced to a variation in the working regime of the impeller 7.

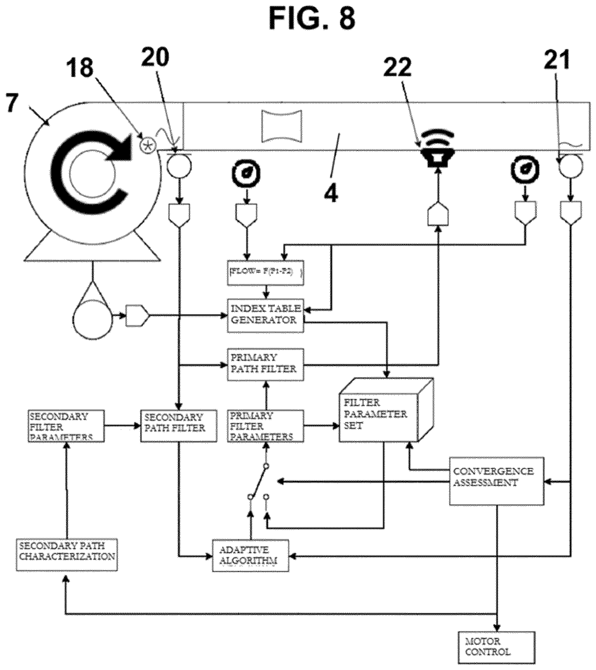

Said electronic system is shown in figure 8, comprising a main microphone 20, an error microphone 21 and a control speaker 22.

The sound from the main microphone 20 is filtered through a series of parameters and characterization provided from a control. Furthermore, the filtered sound and the sound from the error microphone 21 are applied an adaptive algorithm, which is known in the art, providing a signal to the control speaker 22 for active noise cancellation.

To improve active noise cancellation, one or more vibration measurement elements can be included to subtract the parasitic components of the solid vibration from the useful signal present in the air and measured by the microphones 20, 21 of the electronic system of active cancellation.

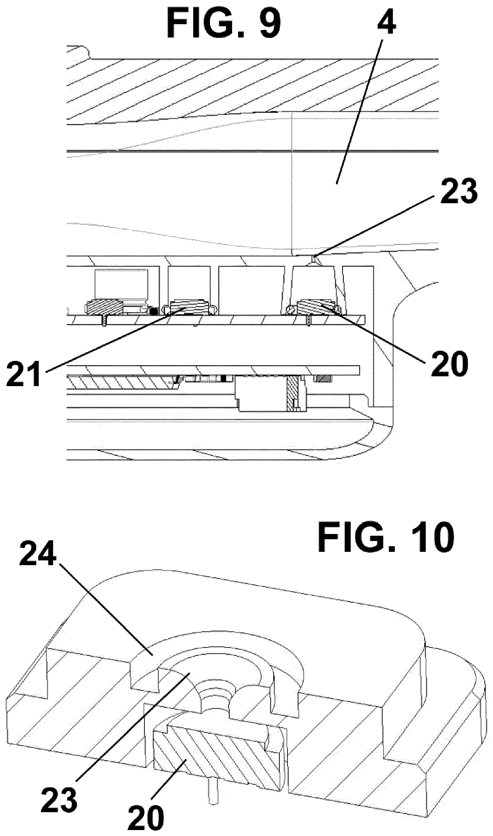

As shown in figure 9, one of these microphones 20 has a hole 23 in communication with channel 4, which captures the sound of channel 4 and the vibration of the housing 1, while the other microphone 21 only captures the vibration of housing 1.

If desired, hard, porous surfaces can be included around the noise pick-up microphones 20, 21 to reduce the acoustic effect of airflow in the vicinity of the pick-up point of the microphone 20, 21.

Auditory panels can also be included at the pickup point of the microphones 20, to move away the turbulence points from the area of auscultation, and also a mechanical isolation of the vibrations.

As shown in Fig. 10, a circular groove 24 may be formed arranged around the hole 23 of the microphone 20, which is funnel-shaped.

If desired, ultraviolet light can also be used inside the air channel 4 for disinfection and the use of an active Helmholtz cavity (with speaker).

Although reference has been made to a specific embodiment of the invention, it is clear to an expert in the field that the described driving device is susceptible to numerous variations and modifications, and that all the mentioned details can be replaced by other technically equivalent ones, without departing from the scope of protection defined by the appended claims.

## Claims

1. Driving device for providing assisted ventilation, comprising an air inlet (15), an impeller (7) provided with blades (8) rotatably driven by a motor (6), and an air outlet (5), the rotation of said impeller (7) being caused by the air circulation through a duct (4) from the air inlet (15) to the air outlet (5), **characterized in that** said blades (8) of the impeller (7) are flexible, vibrating at least one of their ends by the thrust caused by said circulating air.

2. Device according to claim 1, wherein said blades (8) are curved.

3. Device according to claim 1, wherein the end of each blade (8) closest to the axis of rotation of the impeller (7) is fixed with respect to the impeller (7) and the end of each blade (8) furthest from the rotation axis of the impeller (7) is the end that moves due to the vibration caused by the circulating air.

4. Device according to claim 1, which also comprises accelerometers mounted at a point close to the motor attachment (6) and / or on the impeller (7).

5. Device according to claim 1, which also comprises a motor angle gauge (6), which measures the angle of inclination of the motor (6) during the rotation of the impeller (7).

6. Device according to claim 1, wherein said duct (4) comprises a plurality of internal cavities (12).

7. Device according to claim 6, wherein each internal cavity (12) comprises at least one support column (13).

8. Device according to claim 1, wherein said air inlet (15) comprises a plurality of air directing vanes (16).

9. Device according to claim 8, wherein s each of said vanes (16) has a wavy shape.

10. Device according to claim 1, wherein said duct (4) comprises a partition (17) to produce vibrations induced by vortexes.

11. Device according to claim 10, wherein said partition (17) is placed at the end of the duct (4) closest to the air outlet (5).

12. Device according to claim 1, which also comprises a first microphone (20) to detect the sound produced by the air inside the channel (4), said first microphone (20) being located in a housing provided with a hole (23) in communication with the channel (4).

13. Device according to claim 1 or 12, which also comprises a second microphone (21) for detecting the sound produced by the air inside the device, said second microphone (21) being located in a closed housing.

14. Device according to claim 12, wherein said hole (23) is conical in shape.

15. Device according to claim 12 or 14, wherein a circular groove (24) is arranged around said hole (23).
